# EUROPEAN PATENT APPLICATION

(11) **EP 4 566 512 A1**
(43) Date of publication of application: **11.06.2025**
(21) Application number: 23849888.5
(22) Date of filing: 19.07.2023
(51) Int. Cl.: A61B 3/028

(54) **SUBJECTIVE OPTOMETRY DEVICE AND SUBJECTIVE OPTOMETRY PROGRAM**

(30) Priority: 05.08.2022 JP 2022125164
(71) Applicant: Nidek Co., Ltd., Gamagori-shi, Aichi, 443-0038 (JP)
(72) Inventor: TERABE, Hirohisa, Gamagori-shi Aichi 443-0038 (JP); HORINO, Taeko, Gamagori-shi Aichi 443-0038 (JP)
(74) Representative: Hoefer & Partner Patentanwälte mbB
(86) International application number: PCT/JP2023/026343
(87) International publication number: WO 2024/029332

(57) **Abstract**

A subjective optometry device subjectively measures an eye refractive power of a subject eye. The subjective optometry device includes a correction means arranged in front of the subject eye and configured to change an optical characteristic of a target light flux emitted from a target presentation means, and a control means which controls the correction means. The correction means includes a first correction means which changes a spherical refractive power of the target light flux using a variable focus member which has a variable focal length, and a second correction means which switches an optical member to arrange another optical member for changing the spherical refractive power of the target light flux. The control means controls the first correction means and the second correction means to change the spherical refractive power of the target light flux.

## Description

### TECHNICAL FIELD

The present disclosure relates to a subjective optometry device and a subjective optometry program for subjectively measuring an eye refractive power of a subject eye.

### BACKGROUND ART

There is known a subjective optometry device that measures an eye refractive power or the like of a subject eye by arranging an optical member in front of eyes of an examinee and presenting an examination visual target to the subject eye through the optical member. In Patent Literature 1, a variable focus lens capable of changing a focal length is used as an optical member to change a spherical refractive power of a target light flux, thereby changing a spherical correction amount for correcting a subject eye.

### CITATION LIST

### PATENT LITERATURE

Patent Literature 1: JP2017-510384A

### SUMMARY OF INVENTION

For example, the subjective optometry device can change the spherical correction amount of the subject eye seamlessly (without seams) from a certain correction amount to another correction amount in a range (a variable range) in which the spherical refractive power of the target light flux can be changed by the variable focus lens. However, since it is technically difficult to greatly change the focal length of the variable focus lens (that is, to change the spherical refractive power of the target light flux to a high level), when the spherical correction amount of the subject eye exceeds the variable range of the variable focus lens, seamless handling may be difficult.

The present disclosure provides a subjective optometry device and a subjective optometry program capable of accurately measuring an eye refractive power of a subject eye, in view of the prior art.

A subjective optometry device according to a first aspect of the present disclosure is a subjective optometry device for subjectively measuring an eye refractive power of a subject eye, the subjective optometry device including:
a correction means arranged in front of the subject eye, and configured to change an optical characteristic of a target light flux emitted from a target presentation means; and
a control means configured to control the correction means,
in which the correction means includes a first correction means configured to change a spherical refractive power of the target light flux using a variable focus member which has a variable focal length, and a second correction means configured to switch an optical member to arrange another optical member for changing the spherical refractive power of the target light flux, and
the control means controls the first correction means and the second correction means to change the spherical refractive power of the target light flux.

A subjective optometry program according to a second aspect of the present disclosure is a subjective optometry program used in a subjective optometry device for subjectively measuring an eye refractive power of a subject eye, the subjective optometry device including a correction means that is arranged in front of the subject eye and configured to change an optical characteristic of a target light flux emitted from a target presentation means, and includes a first correction means configured to change a spherical refractive power of the target light flux using a variable focus member which has a variable focal length, and a second correction means configured to switch an optical member to arrange another optical member for changing the spherical refractive power of the target light flux,
the subjective optometry program including an instruction which, when executed by a processor of the subjective optometry device, causes the subjective optometry device to perform:
a control step of controlling the correction means,
in which in the control step, the first correction means and the second correction means are controlled to change the spherical refractive power of the target light flux.

### BRIEF DESCRIPTION OF DRAWINGS

[FIG. 1A] FIG. 1A is an external view of an eye refractive power measurement unit supported at a standby position.
[FIG. 1B] FIG. 1B is an external view of an eye refractive power measurement unit supported at a measurement position.
[FIG. 2A] FIG. 2A is a schematic diagram illustrating optical arrangement at the time of a distant vision examination of a light projecting optical system.
[FIG. 2B] FIG. 2B is a schematic diagram illustrating optical arrangement at the time of a near vision examination of the light projecting optical system.
[FIG. 3] FIG. 3 is a schematic diagram of the eye refractive power measurement unit.
[FIG. 4A] FIG. 4A is an internal configuration diagram of a lens unit.
[FIG. 4B] FIG. 4B is a horizontal sectional view of the lens unit.
[FIG. 5A] FIG. 5A is an internal configuration diagram of a lens unit in related art.
[FIG. 5B] FIG. 5B is a horizontal sectional view of the lens unit in related art.
[FIG. 6] FIG. 6 illustrates a change in a synthetic refractive power of a cylindrical lens of a cylindrical lens disk and a cross cylinder lens.
[FIG. 7] FIG. 7 is a schematic diagram of a control system of a subjective optometry device.
[FIG. 8] FIG. 8 illustrates a change in a synthetic refractive power of a cylindrical lens of a cylindrical lens disk and a cross cylinder lens.

### DESCRIPTION OF EMBODIMENTS

### <Overview>

An overview of a subjective optometry device according to an embodiment of the present disclosure will be described. In the present embodiment, a left-right direction of the subjective optometry device is an X direction, an up-down direction is a Y direction, and a front-rear direction (a working distance direction) is a Z direction. The letters L and R attached to the reference numerals indicate the left eye and the right eye, respectively. Items classified as the following sign "< >" may be used independently of or in relation to each other.

The subjective optometry device (for example, a subjective optometry device 100) according to the present embodiment is a device for subjectively measuring an eye refractive power of a subject eye. For example, at least any one of a spherical eye refractive power, a cylindrical eye refractive power, an astigmatic axis angle, and the like may be measured as the eye refractive power of the subject eye. Of course, the subjective optometry device may measure, in addition to the eye refractive power, a binocular vision function (for example, at least any one of a prism amount, a stereoscopic function, and the like), contrast sensitivity, and the like.

The subjective optometry device according to the present embodiment includes, for example, a configuration including a target presentation means and a correction means to be described later, but is not limited thereto. The subjective optometry device may be configured to include at least a correction means. For example, the subjective optometry device may include only a correction means, or may include the target presentation means and the correction means as a system.

The subjective optometry device according to the present embodiment may include the target presentation means. The target presentation means emits a target light flux toward the subject eye.

For example, the target presentation means may be a display (for example, a display 31). For example, the target presentation means may be a light source and a digital micromirror device (DMD). For example, the target presentation means may be a light source and a visual target plate.

For example, the target light flux from the target presentation means may be directly guided toward the subject eye. For example, the target light flux emitted from the target presentation means may be guided toward the subject eye via a light projecting optical system (for example, a light projecting optical system 30). For example, the light projecting optical system may include at least one optical member for passing the target light flux emitted from the target presentation means. The light projecting optical system may include at least any one of a lens, a mirror, and the like as an example.

The subjective optometry device according to the present embodiment may include a correction means. The correction means is arranged in front of the subject eye, and changes optical characteristics of the target light flux emitted from the target presentation means.

The correction means may be configured to be able to change at least any one of the optical characteristics of the target light flux, such as a spherical refractive power, a cylindrical refractive power, and an astigmatic axis angle. As an example, the correction means may be an eye refractive power measurement unit (for example, an eye refractive power measurement unit 40) which switches an optical member (for example, an optical element 51) to arrange another optical member via an examination window (for example, an examination window 43) in front of the subject eye. For example, the optical member may be at least any one of a spherical lens, a cylindrical lens, a variable focus lens, a cross cylinder lens, a rotary prism, a wavefront modulation element, and the like. Of course, the optical member may be different from the above. For example, the eye refractive power measurement unit may include a lens disk (for example, a lens disk 50) in which a plurality of optical members are arranged on the same circumference. In this case, the optical characteristics of the target light flux are changed by controlling a drive means (for example, a drive unit 52, a drive unit 53, and the like) for controlling the lens disk.

### <Change in Spherical Refractive Power of Target Light Flux>

In the present embodiment, the correction means may include a first correction means which changes the spherical refractive power of the target light flux using a variable optical member which has a variable focal length, and a second correction means which switches the optical member to arrange another optical member for changing the spherical refractive power of the target light flux.

The first correction means is configured to change the spherical refractive power of the target light flux by changing a focal length of a variable focus member in a state in which the variable focus member is arranged in an optical path of the target light flux. For example, the first correction means may switch the variable focus member to another variable focus member in front of the subject eye. As an example, in this case, the variable focus member may be provided on the lens disk of the eye refractive power measurement unit. For example, the first correction means may fixedly arrange the variable focus member in front of the subject eye. As an example, in this case, the variable focus member may be normally arranged in the examination window of the eye refractive power measurement unit. For example, the number of variable focus members may be one or more. For example, the variable focus member may be a variable focus lens. As the variable focus lens, at least any one of a liquid lens, a liquid crystal lens, an Alvarez lens, and the like can be used.

The first correction means may change the spherical refractive power of the target light flux within a range of a first refractive power using the variable focus member. That is, the spherical refractive power of the target light flux may be continuously changed by using the variable focus member.

The second correction means is configured to change the spherical refractive power of the target light flux by switching between the optical members to be arranged in the optical path of the target light flux. For example, the second correction means may switch the optical member to arrange another optical member in front of the subject eye. As an example, in this case, the optical member may be provided on the lens disk of the eye refractive power measurement unit. For example, the number of second optical elements may be one or more. For example, the optical member may be a spherical lens. When the variable focus member is used as the optical member of the second correction means, the focal length of the variable focus member may be set to a fixed distance.

A step in which the first correction means enables to change the spherical refractive power of the target light flux may be smaller than a step in which the second correction means enables to change the spherical refractive power of the target light flux. For example, the spherical refractive power of the target light flux may be changed in units of 0.25 D or less in the first correction means, and the spherical refractive power of the target light flux may be changed in units larger than 0.25 D in the second correction means. Of course, the value of the step of the spherical refractive power is an example, and may be different. Accordingly, the spherical refractive power of the target light flux can be continuously changed within the range of the first refractive power using the first correction means. In addition, by using the first correction means and the second correction means in combination, it is possible to enlarge the spherical refractive power of the target light flux to a range of a synthetic spherical refractive power which is larger than the first refractive power, and it is possible to continuously change the spherical refractive power of the target light flux in the range of the synthetic refractive power.

The subjective optometry device according to the present embodiment may include a determination means (for example, a control unit 70). The determination means determines whether or not the optical member of the second correction means is arranged in front of the subject eye, based on the changed spherical refractive power obtained by changing the spherical refractive power of the target light flux. In other words, it is determined whether or not the optical member of the second correction means is arranged in front of the subject eye, based on a spherical correction amount for correcting the subject eye that can be changed by changing the spherical refractive power of the target light flux. For example, the determination means may determine whether or not the optical member is arranged, based on whether or not the changed spherical refractive power of the target light flux exceeds a predetermined threshold value set in advance. For example, the predetermined threshold value may be a fixed value or any value that may be set by an examiner. Accordingly, the variable focus member of the first correction means and the optical member of the second correction means can be appropriately combined as necessary.

The determination means may determine whether or not the optical member of the second correction means is arranged in front of the subject eye, based on the changed spherical refractive power of the target light flux and the range of the first refractive power of the first correction means. In this case, the threshold value of the changed spherical refractive power of the target light flux may be set based on the range of the first refractive power of the first correction means. For example, the predetermined threshold value may be a range from a maximum value to a minimum value in the first refractive power of the first correction means. For example, the predetermined threshold value may be a range from a value around the maximum value in the first refractive power of the first correction means to a value around the minimum value. As an example, the predetermined threshold value may be a range from a value smaller than the maximum value of the first refractive power by one step to a value larger than the minimum value by one step. Accordingly, a case that cannot be coped only by adjusting the spherical refractive power of the target light flux using the first correction means, such as a case where the spherical correction amount of the subject eye is a high power, can be easily grasped, and the variable focus member of the first correction means and the optical member of the second correction means can be appropriately combined.

The subjective optometry device according to the present embodiment may include a control means (for example, the control unit 70). The control means controls the correction means. For example, the control means controls the first correction means and the second correction means and changes the spherical refractive power of the target light flux. For example, by changing the spherical refractive power of the target light flux, the spherical correction amount for correcting the subject eye is changed. Accordingly, since the spherical refractive power of the target light flux varies seamlessly (without seams), a sense of discomfort caused by switching the spherical refractive power of the target light flux from a first changed spherical refractive power to a second changed spherical refractive power different from the first changed spherical refractive power (in other words, switching the spherical correction amount of the subject eye from a first spherical correction amount to a second spherical correction amount) can be reduced, and the eye refractive power of the subject eye can be accurately measured.

The control means may change the spherical refractive power of the target light flux and change the spherical correction amount of the subject eye by arranging the variable focus member of the first correction means in the optical path of the target light flux and changing the focal length of the variable focus member. For example, the control means may rotate the lens disk of the eye refractive power measurement unit to arrange the variable focus member on the lens disk in the examination window (that is, in the optical path of the target light flux). For example, the control means may change the focal length of the variable focus member in the examination window of the eye refractive power measurement unit.

The control means may change the spherical refractive power of the target light flux and change the spherical correction amount of the subject eye by arranging a predetermined optical member of the second correction means in the optical path of the target light flux. For example, the control means may rotate the lens disk of the eye refractive power measurement unit to arrange the optical member on the lens disk in the examination window (that is, in the optical path of the target light flux).

For example, when the spherical refractive power of the target light flux is changed by the variable focus member of the first correction means, the variable focus member may be changed to a predetermined focal length in a state in which the variable focus member of the first correction means is arranged in the optical path of the target light flux and the optical member of the second correction means is removed from the optical path of the target light flux. For example, when the spherical refractive power of the target light flux is changed by the optical member of the second correction means, the variable focus member of the first correction means may be removed from the optical path of the target light flux, and the optical member of the second correction means may be arranged in the optical path of the target light flux. Alternatively, the variable focus member may be changed to a focal length of 0 D in a state in which both the variable focus member of the first correction means and the optical member of the second correction means may be arranged in the optical path of the target light flux.

Of course, for example, when the spherical refractive power of the target light flux is changed by the combination of the variable focus member of the first correction means and the optical member of the second correction means, the variable focus member may be changed to the predetermined focal length in a state in which both the variable focus member and the optical member are arranged in the optical path of the target light flux. By combining the variable focus member and the optical member, it is possible to greatly change the spherical refractive power of the target light flux. Therefore, even when the spherical correction amount of the subject eye is large, the eye refractive power of the subject eye can be accurately measured.

The control means may arrange at least the optical member of the second correction means based on the changed spherical refractive power obtained by changing the spherical refractive power of the target light flux. That is, the control means may arrange only the optical member of the second correction means based on the changed spherical refractive power of the target light flux, or may adjust the variable focus member of the first correction means and arrange the optical member of the second correction means.

For example, in a case where the changed spherical refractive power of the target light flux exceeds the range of the first refractive power in the first correction means, the control means may control at least the second correction means to switch the optical member to arrange another optical member. In other words, in at least any one of a case where the changed spherical refractive power of the target light flux exceeds the maximum value of the first refractive power of the first correction means and a case where the changed spherical refractive power of the target light flux exceeds the minimum value of the first refractive power, at least the optical member may be arranged. In addition, for example, the control means may arrange at least the optical member in at least any one of a case where the changed spherical refractive power of the target light flux is within the range of the first refractive power of the spherical refractive power in the first correction means and exceeds the value around the maximum value of the first refractive power and a case where the changed spherical refractive power of the target light flux exceeds the value around the minimum value of the first refractive power.

The control means may adjust the variable focus member of the first correction means using a table or an arithmetic expression in which the changed spherical refractive power of the target light flux and the first refractive power of the first correction means are associated with each other. Further, the control means may set whether or not to arrange the optical member of the second correction means using a table or an arithmetic expression in which the changed spherical refractive power of the target light flux and the second refractive power of the second correction means are associated with each other. Of course, the control means may execute the adjustment of the variable focus member of the first correction means and the setting of whether or not to arrange the optical member of the second correction means using a table or an arithmetic expression in which the changed spherical refractive power of the target light flux, the first refractive power of the first correction means, and the second refractive power of the second correction means are associated with each other. For example, such a table may be obtained in advance by experiments or simulations and stored in a storage means.

The control means may control at least the second correction means based on a determination result of the determination means such that the optical member is switched to arrange another optical member. For example, when a determination result that the change amount in the spherical refractive power of the target light flux does not exceed the predetermined threshold value is obtained, the control means may control the first correction means and generate a predetermined spherical refractive power using the variable focus member. For example, when a determination result that the change amount in the spherical refractive power of the target light flux exceeds the predetermined threshold value is obtained, the control means may control at least the second correction means and generate a predetermined spherical refractive power using at least the optical member. According to the change amount in the spherical refractive power of the target light flux, both the first correction means and the second correction means may be controlled, and a predetermined spherical refractive power may be generated using the variable focus member and the optical member. Accordingly, the eye refractive power of the subject eye can be accurately measured.

In a case where the changed spherical refractive power of the target light flux is adjusted from the first changed spherical refractive power to the second changed spherical refractive power different from the first changed spherical refractive power, the control means may control only the first correction means according to a change amount between the first changed spherical refractive power and the second changed spherical refractive power, to change the focal length of the variable focus member. For example, when the change amount between the first changed spherical refractive power before the spherical refractive power of the target light flux is changed and the second changed spherical refractive power after the change is equal to or smaller than the predetermined threshold value, only the first correction means may be controlled. For example, when the change amount between the first changed spherical refractive power and the second changed spherical refractive power exceeds the predetermined threshold value, both the first correction means and the second correction means, or only the second correction means may be controlled. For example, the predetermined threshold value may be a fixed value or any value that may be set by an examiner. Accordingly, it is possible to easily adjust a spherical correction power necessary for the correction of the subject eye and smoothly measure the eye refractive power of the subject eye.

### <Change in Cylindrical Refractive Power and Astigmatic Axis Angle of Target Light Flux>

In the present embodiment, the correction means may include a Stokes lens including a first cylindrical lens and a second cylindrical lens which are independently rotatable in front of the subject eye. For example, the correction means may continuously change the cylindrical refractive power of the target light flux by respectively rotating the first cylindrical lens and the second cylindrical lens and changing a relative angle of an astigmatic axis of each cylindrical lens. For example, the correction means may continuously change the astigmatic axis angle of the target light flux by integrally rotating the first cylindrical lens and the second cylindrical lens and changing a synthetic axis angle of each cylindrical lens. For example, the first cylindrical lens and the second cylindrical lens may be configured by two positive cylindrical lenses having the same focal length, or may be configured by a positive cylindrical lens and a negative cylindrical lens having the same focal length.

The correction means may include a compensation optical member for compensating a deviation amount of the spherical correction amount of the subject eye, that is, a deviation amount of the spherical correction amount caused by aligning the first cylindrical lens and the second cylindrical lens with the synthetic axis angle. For example, the compensation optical member may be a member capable of changing the spherical refractive power of the target light flux. As an example, at least any one of a variable focus member having a variable focal length and an optical member having a fixed focal length may be used. For example, the number of the compensation optical members may be one or more.

For example, the correction means may switch the compensation optical member to arrange another compensation optical member in front of the subject eye. As an example, in this case, the compensation optical member may be provided on the lens disk of the eye refractive power measurement unit. Such a compensation optical member may be at least any one of a variable focus member (for example, a variable focus lens) and an optical member (for example, a spherical lens). For example, the correction means may fixedly arrange the compensation optical member in front of the subject eye. As an example, in this case, the compensation optical member may be normally arranged in the examination window of the eye refractive power measurement unit. Such a compensation optical member may be a variable focus member (for example, a variable focus lens).

The correction means may include a correction optical member for correcting the spherical correction amount of the subject eye. For example, the correction optical member may be a member capable of changing the spherical refractive power of the target light flux. As an example, at least any one of a variable focus member having a variable focal length and an optical member having a fixed focal length may be used. For example, the number of the correction optical members may be one or more.

For example, the correction means may switch the correction optical member to arrange another correction optical member in front of the subject eye. As an example, in this case, the correction optical member may be provided on the lens disk of the eye refractive power measurement unit. Such a correction optical member may be at least any one of a variable focus member (for example, a variable focus lens) and an optical member (for example, a spherical lens). For example, the correction means may fixedly arrange the correction optical member in front of the subject eye. As an example, in this case, the correction optical member may be normally arranged in the examination window of the eye refractive power measurement unit. Such a correction optical member may be a variable focus member (for example, a variable focus lens).

In the present embodiment, the compensation optical member for compensating the deviation amount of the spherical correction amount caused by aligning the first cylindrical lens and the second cylindrical lens included in the Stokes lens with the synthetic axis angle and the correction optical member for adjusting the spherical correction amount for correcting the subject eye may be used as both.

Further, in the present embodiment, the compensation optical member for compensating the deviation amount of the spherical correction amount of the subject eye and the variable focus member having a variable focal length of the first correction means may be shared, or the compensation optical member for compensating the deviation amount of the spherical correction amount of the subject eye and the optical member of the second correction means may be used as both. Similarly, in the present embodiment, the correction optical member for adjusting the spherical correction amount of the subject eye and the variable focus member having a variable focal length of the first correction means may be used as both, or the correction optical member for adjusting the spherical correction amount of the subject eye and the optical member of the second correction means may be used as both. Accordingly, the spherical correction amount of the subject eye can be easily adjusted.

The subjective optometry device according to the present embodiment may include an acquisition means (for example, the control unit 70). The acquisition means acquires at least a cylindrical correction amount and an astigmatic axis correction amount for the subject eye. For example, the acquisition means may acquire the cylindrical correction amount and the astigmatic axis correction amount based on a refractive power (an objective value) obtained by objectively measuring the subject eye. In addition, for example, the acquisition means may acquire the cylindrical correction amount and the astigmatic axis correction amount based on a refractive power (a subjective value) obtained by subjectively measuring the subject eye. As an example, the subjective value of the subject eye may be a most positive corrected refractive power (a best correction value) at which a maximum visual acuity of the subject eye is obtained, a corrected refractive power (a prescription value) at which predetermined visual acuity of the subject eye is obtained, or the like. Of course, the acquisition means may acquire the spherical correction amount of the subject eye together with the cylindrical eye refractive power and the astigmatic axis angle of the subject eye.

For example, the acquisition means may acquire a cylindrical correction amount and an astigmatic axis correction amount input by operating an operation means (for example, an examiner controller 10) by the examiner. For example, the acquisition means may read an identifier for each examinee and acquire a cylindrical correction amount and an astigmatic axis correction amount stored in the identifier. As an example, an ID, a character string, a one-dimensional code, a two-dimensional code, a color code, or the like may be used as the identifier. For example, the acquisition means may acquire the cylindrical correction amount and the astigmatic axis correction amount by receiving data measured using a device different from the subjective optometry device of the present embodiment.

The subjective optometry device according to the present embodiment may include an addition power acquisition means (for example, the control unit 70). The addition power acquisition means acquires an addition power to a correction amount at a predetermined examination distance of the subject eye. For example, the addition power may be a power based on at least any one of a refractive power, an accommodative power, an age, and the like of the subject eye.

For example, the addition power acquisition means may acquire an addition power input by operating an operation means by the examiner. For example, the addition power acquisition means may read an identifier of each examinee and acquire an addition power stored in the identifier. For example, the addition power acquisition means may acquire the addition power by receiving data measured using a device different from the subjective optometry device of the present embodiment.

The subjective optometry device according to the present embodiment may include a control means. Note that the control means can use the same control means as that described in <Change in Spherical Refractive Power of Target Light Flux>. Of course, different control means may be separately provided.

The control means may control the correction means in the addition power examination for measuring the addition power to the correction amount at a predetermined examination distance of the subject eye. In other words, the control means may control the Stokes lens in the addition power examination. For example, the control means may change the synthetic axis angle of the first cylindrical lens and the second cylindrical lens to a predetermined axis angle based on the cylindrical correction amount and the astigmatic axis correction amount acquired by the acquisition means. For example, such a synthetic axis angle may be an axis angle in consideration of the deviation amount of the astigmatic axis angle that occurs when the optical member and the cross cylinder lens are assumed to be arranged in order to correct the subject eye with the cylindrical correction amount and the astigmatic axis correction amount. The optical member here may be an optical member (for example, a cylindrical lens) capable of adjusting at least any one of the cylindrical correction amount and the astigmatic axis correction amount of the subject eye by changing at least any one of the cylindrical refractive power and the astigmatic axis angle of the target light flux. Accordingly, since the cylindrical refractive power and the astigmatic axis angle of the target light flux vary seamlessly (without seams), a sense of discomfort caused by switching the cylindrical refractive power of the target light flux from a first changed cylindrical refractive power to a second changed cylindrical refractive power different from the first changed cylindrical refractive power (in other words, switching the cylindrical correction amount of the subject eye from a first cylindrical correction amount to a second cylindrical correction amount) can be reduced, and the eye refractive power of the subject eye can be accurately measured. Similarly, a sense of discomfort caused by switching the astigmatic axis angle of the target light flux from the first changed astigmatic axis angle to a second changed astigmatic axis angle different from the first changed astigmatic axis angle (in other words, switching the astigmatic axis correction amount of the subject eye from a first astigmatic axis correction amount to a second astigmatic axis correction amount) can be reduced, and the eye refractive power of the subject eye can be accurately measured.

The control means may compensate the deviation amount of the spherical correction amount of the subject eye by arranging the compensation optical member in front of the subject eye. In other words, the deviation amount of the spherical correction amount of the subject eye may be compensated by arranging the compensation optical member in the optical path of the target light flux and changing the spherical refractive power of the target light flux. For example, the control means may rotate the lens disk of the eye refractive power measurement unit to arrange the variable focus member on the lens disk as the compensation optical member on the examination window, and may change the focal length of the variable focus member. For example, the control means may rotate the lens disk of the eye refractive power measurement unit to arrange the optical member on the lens disk as the compensation optical member on the examination window. Accordingly, the spherical correction amount of the subject eye is appropriately compensated by the compensation optical member.

The control means may compensate the deviation amount of the spherical correction amount of the subject eye by changing the spherical refractive power in the variable focus member using the variable focus member which has a variable focal length as the compensation optical member. For example, since the deviation amount of the spherical correction amount due to the adjustment of the Stokes lens is small, the accuracy of compensation is further improved by finely adjusting the deviation amount using the variable focus member.

The control means may set the synthetic axis angle using a table or an arithmetic expression in which the cylindrical correction amount and the astigmatic axis correction amount of the subject eye are associated with the synthetic axis angle of the first cylindrical lens and the second cylindrical lens. In addition, the control means may arrange the compensation optical member using a table or an arithmetic expression in which the cylindrical correction amount and an astigmatism correction amount of the subject eye are associated with a deviation amount of an astigmatism axial angle correction amount according to the setting of the synthetic axis angle of the first cylindrical lens and the second cylindrical lens. Of course, the control means may execute the setting of the synthetic axis angle and the arrangement of the compensation optical member using a table or an arithmetic expression in which the cylindrical correction amount and the astigmatic axis correction amount of the subject eye, the synthetic axis angle of the first cylindrical lens and the second cylindrical lens, and the deviation amount of the astigmatic axis correction amount according to the setting of the synthetic axis angle are associated with each other. For example, such a table may be obtained in advance by experiments or simulations and stored in a storage means.

The control means may switch between a first state in which the first cylindrical lens and the second cylindrical lens are aligned with the synthetic axis angle and a second state in which the correction optical member based on the addition power is further arranged in the first state. For example, the control means may switch from the first state to the second state by switching the correction optical member to arrange another correction optical member in the optical path of the target light flux for changing the spherical refractive power of the target light flux. For example, the control means may rotate the lens disk of the eye refractive power measurement unit to arrange the variable focus member on the lens disk as the correction optical member on the examination window, and may change the focal length of the variable focus member. For example, the control means may rotate the lens disk of the eye refractive power measurement unit to arrange the optical member on the lens disk as the correction optical member on the examination window. Accordingly, the spherical correction amount of the subject eye is appropriately corrected by the correction optical member. In a state in which the deviation amount of the spherical correction amount of the subject eye is compensated, a state in which the addition power is not applied to the subject eye and a state in which the addition power is applied to the subject eye are switched, so that an optimum addition power for the subject eye can be accurately measured.

The present disclosure is not limited to the device described in the present embodiment. For example, terminal control software (a program) that performs functions of the above-described embodiment may be supplied to an apparatus or a system via a network or various storage media, and a control device (for example, a CPU or the like) of the apparatus or the system may read and execute the program.

### <Example>

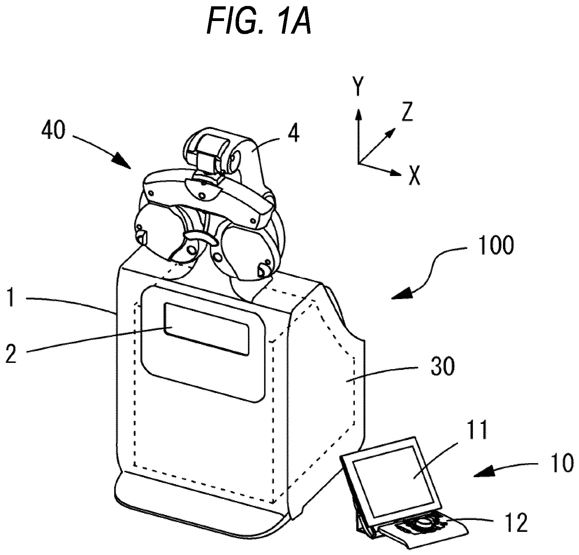

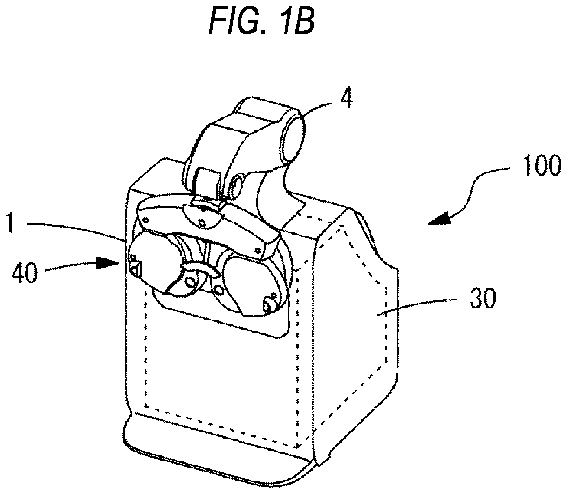

An example of the subjective optometry device according to the present embodiment will be described. FIGS. 1A and 1B are external views of the subjective optometry device 100. FIG. 1A shows a state in which the eye refractive power measurement unit 40 is supported at a standby position. FIG. 1B shows a state in which the eye refractive power measurement unit 40 is supported at a measurement position. For example, the subjective optometry device 100 includes a housing 1, a presentation window 2, a holding unit 4, an examiner controller 10, and the eye refractive power measurement unit 40.

The housing 1 has the light projecting optical system 30 therein. The presentation window 2 transmits a target light flux by the light projecting optical system 30. The target light flux is projected on a subject eye E through the presentation window 2. When the eye refractive power measurement unit 40 is arranged between the subject eye E and the presentation window 2 (see FIG. 1B), the target light flux is projected on the subject eye E through the presentation window 2 and the examination window 43 to be described later. Accordingly, an examination visual target is presented on the subject eye E.

The holding unit 4 holds the eye refractive power measurement unit 40. For example, the holding unit 4 moves an arm by driving a drive unit (such as a motor) (not shown) to move the eye refractive power measurement unit 40 connected to the arm. Accordingly, the standby position and the measurement position of the eye refractive power measurement unit 40 are switched.

The examiner controller 10 is used by an examiner to operate the subjective optometry device 100. The examiner controller 10 includes a switch unit 11, a monitor 12, and the like. The switch unit 11 inputs signals for performing various settings (for example, selection of visual targets to be presented to an examinee). The monitor 12 displays various types of information (for example, measurement results of the subject eye E, or the like). The monitor 12 may function as a touch panel doubling as the switch unit 11. A signal from the examiner controller 10 is output to a control unit 60 by wired communication or wireless communication.

### <Light Projecting Optical System>

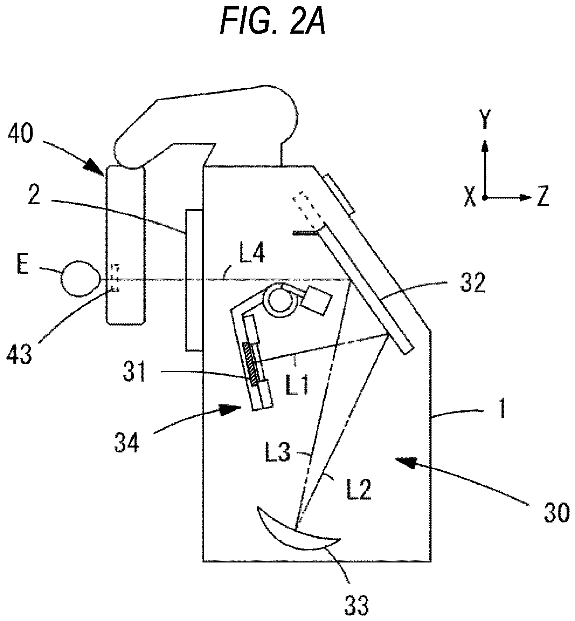

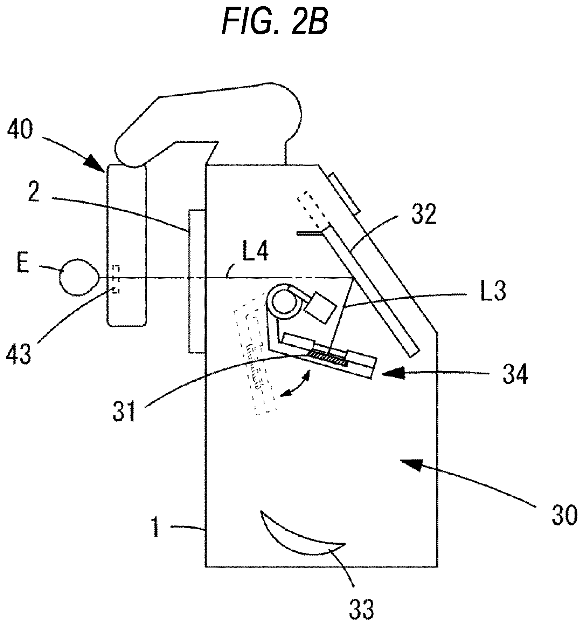

FIGS. 2A and 2B are schematic diagrams of the light projecting optical system 30. FIG. 2A shows optical arrangement at the time of a distant vision examination. FIG. 2B shows optical arrangement at the time of a near vision examination. The light projecting optical system 30 emits the target light flux toward the subject eye E. For example, the light projecting optical system 30 includes a display 31, a plane mirror 32, a concave mirror 33, a distant-near switching unit 34, and the like.

The display 31 displays a visual target (for example, a fixation target, an examination visual target, or the like). When the target light flux emitted from the display 31 is imaged on a fundus of the subject eye E, the visual target is presented on the subject eye E. For example, the display 31 may be a liquid crystal display (LCD), an organic electro luminescence (EL), a plasma display, or the like.

The plane mirror 32 reflects the target light flux from the display 31 and guides the target light flux to the concave mirror 33. The plane mirror 32 reflects the target light flux from the display 31 and guides the target light flux to the subject eye E. For example, the plane mirror 32 is arranged such that a distance (a presentation distance) from the subject eye E to the display 31 is optically 40 cm at the time of the near vision examination for the subject eye E. Instead of the plane mirror 32, a reflection member such as a prism, a beam splitter, or a half mirror may be used.

The concave mirror 33 reflects the target light flux from the display 31 and guides the target light flux to the plane mirror 32. For example, the concave mirror 33 is arranged such that the distance (the presentation distance) from the subject eye E to the display 31 is optically 5m at the time of the distant vision examination for the subject eye E. Instead of the concave mirror 33, a reflection member such as an aspherical mirror or a free curved surface mirror may be used. A lens or the like may be used instead of the concave mirror 33.

The distant-near switching unit 34 switches the arrangement of the display 31 at the time of the distant vision examination and the near vision examination for the subject eye E. For example, the distant-near switching unit 34 moves the display 31 held by the holding unit by moving the holding unit by being driven by a drive unit (such as a motor) (not shown). Accordingly, the distant vision arrangement and the near vision arrangement of the display 31 are switched.

For example, at the time of the distant vision examination for the subject eye E, a display screen of the display 31 is directed to a back surface of the housing 1 (see FIG. 2A). The target light flux from the display 31 passes through an optical axis L1 and enters the plane mirror 32, and is reflected in a direction of an optical axis L2 by the plane mirror 32. In addition, the target light flux passes through the optical axis L2 and enters the concave mirror 33, and is reflected in a direction of an optical axis L3 by the concave mirror 33. Further, the target light flux passes through the optical axis L3 and enters the plane mirror 32, and is reflected in a direction of an optical axis L4 by the plane mirror 32. Accordingly, the target light flux emitted to the outside of the housing 1 is projected onto the subject eye E via the respective optical members inside the housing 1.

For example, at the time of the near vision examination for the subject eye E, the display screen of the display 31 is directed to an upper surface of the housing 1 (see FIG. 2B). The target light flux from the display 31 passes through the optical axis L3 and enters the plane mirror 32, and is reflected in the direction of the optical axis L4 by the plane mirror 32. Accordingly, the target light flux emitted to the outside of the housing 1 is projected onto the subject eye E via the respective optical members inside the housing 1.

### <Eye Refractive Power Measurement Unit (Correction Optical System)>

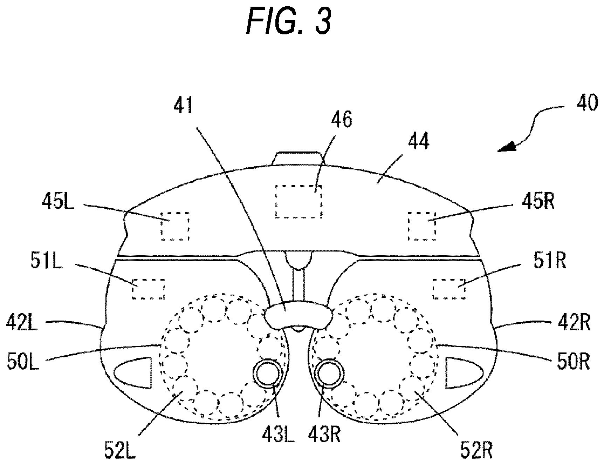

FIG. 3 is a schematic diagram of the eye refractive power measurement unit 40. The eye refractive power measurement unit 40 subjectively measures a refractive power of the subject eye E. The eye refractive power measurement unit 40 is used as a correction optical system. The correction optical system is arranged in an optical path of the light projecting optical system 30 to change optical characteristics of the target light flux. For example, the eye refractive power measurement unit 40 includes a forehead rest 41, a lens unit 42, the examination window 43, a moving unit 44, and the like.

The forehead rest 41 abuts against a forehead of the examinee to fix the subject eye E in a predetermined examination position and keep a distance from the subject eye E to the examination window 43 constant. The lens unit 42 includes a pair of left and right lens units 42L and 42R. The lens unit 42 includes the examination window 43 (a left examination window 43L and a right examination window 43R).

The moving unit 44 adjusts an interval between the left lens unit 42L and the right lens unit 42R and a convergence angle (inward angle) of the left lens unit 42L and the right lens unit 42R. For example, the moving unit 44 adjusts the interval between the left lens unit 42L and the right lens unit 42R by being driven by a drive unit 45 (a left drive unit 45L and a right drive unit 45R). For example, the moving unit 44 adjusts the convergence angle of the left lens unit 42L and the right lens unit 42R by being driven by a drive unit 46. For a detailed configuration of the moving unit 44, for example, refer to JP2004-329345A.

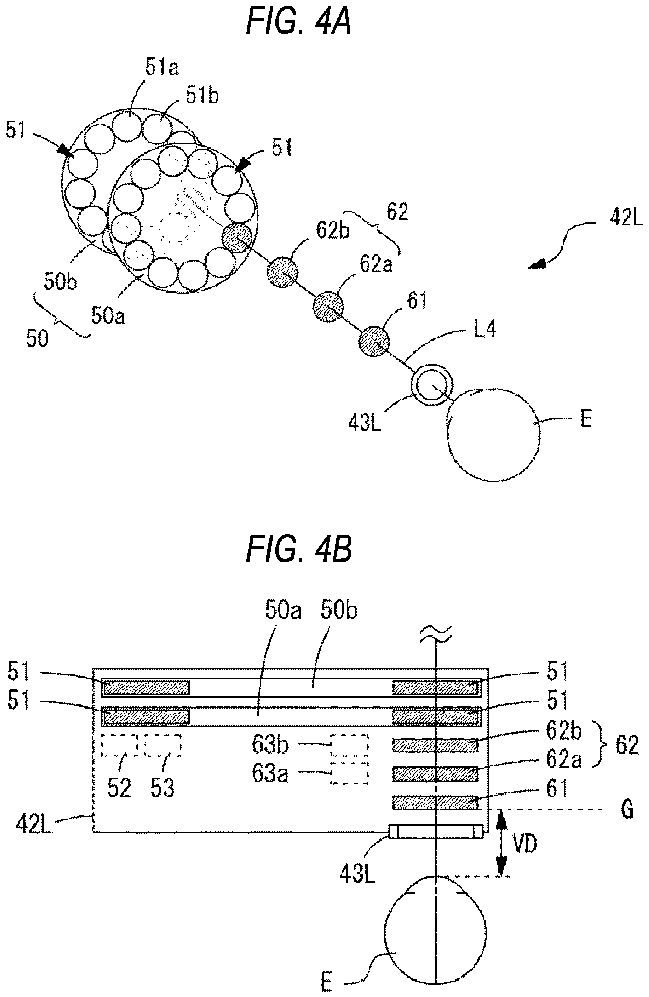

FIGS. 4A and 4B are schematic diagrams of the lens unit 42. FIG. 4A is an internal configuration diagram of the lens unit 42. FIG. 4B is a horizontal sectional view of the lens unit 42. In FIGS. 4A and 4B, only the left lens unit 42L is illustrated, and illustration of the right lens unit 42R is omitted. For example, the lens unit 42 includes a variable focus lens 61, a Stokes lens 62, and a lens disk 50.

The variable focus lens 61 is fixedly arranged in the lens unit 42. The variable focus lens 61 can generate a spherical refractive power that continuously changes within a predetermined range by adjusting the spherical refractive power according to a magnitude of an applied voltage and changing a focal position. For example, in this example, a spherical refractive power of -5.00 D to +5.00 D can be generated.

The Stokes lens 62 is rotatably arranged in the lens unit 42. The Stokes lens 62 includes two cylindrical lenses 62a and 62b. For example, the cylindrical lenses 62a and 62b are a positive cylindrical lens and a negative cylindrical lens having the same focal length. The cylindrical lenses 62a and 62b may be two positive cylindrical lenses having the same focal length.

The cylindrical lens 62a and the cylindrical lens 62b are rotated independently about the optical axis L4 by being driven by rotation mechanisms 63a and 63b, respectively. By changing a rotation angle of at least one of the cylindrical lens 62a and the cylindrical lens 62b and providing a difference in an axis angle of each cylindrical lens, a cylindrical refractive power that continuously changes within a predetermined range can be generated. For example, in this example, a cylindrical refractive power of -10.00 D to +10.00 D can be generated. In addition, by integrally changing the rotation angles of both the cylindrical lens 62a and the cylindrical lens 62b while maintaining the difference in the axis angle thereof (that is, by changing a synthetic axis angle of the cylindrical lens 62a and the cylindrical lens 62b), it is possible to adjust an astigmatic axis angle that continuously changes within a predetermined range. For example, in this example, the astigmatic axis angle can be adjusted from 1 degree to 180 degrees.

The lens disk 50 has an opening (or a lens of 0 D) and a plurality of optical elements 51 on the same circumference. The lens disk 50 is rotated around a disk center by being driven by the drive unit 52. Each of the optical elements 51 is rotated about the optical axis L4 by being driven by the drive unit 53. Accordingly, the desired optical element 51 is switched and arranged in the examination window 43 at a desired angle.

The lens disk 50 includes one lens disk or a plurality of lens disks. For example, in this example, a first auxiliary lens disk 50a and a second auxiliary lens disk 50b are provided. The first auxiliary lens disk 50a is provided with the optical elements 51 such as a polarizing filter, a red filter/green filter, a dispersing prism, a Maddox lens, and the like. The second auxiliary lens disk 50b is provided with the optical elements 51, such as a plain lens, a rotary prism, an auto-cross cylinder lens, a first spherical lens 51a, and a second spherical lens 51b. For example, a mark for aligning an interpupillary distance of the subject eye E is attached to the plain lens. For example, the first spherical lens 51a is a lens having a spherical power of - 10.00 D in terms of a spectacle wearing position. For example, the second spherical lens 51b is a lens having a spherical power of +10.00 D in terms of the spectacle wearing position. For example, the first spherical lens 51a and the second spherical lens 51b each have a spherical refractive power larger than a predetermined range of the spherical refractive power of the variable focus lens 61.

The spectacle wearing position in this example is a position on the assumption that a spectacle lens is arranged in front of the subject eye E when the examinee wears eyeglasses, and is a position of an optical element (that is, the variable focus lens 61) closest to the subject eye E. More specifically, the spectacle wearing position is a position G of a rear surface of the variable focus lens 61. A distance from a cornea apex position of the subject eye E to the position of the variable focus lens 61 (the position G of the rear surface of the variable focus lens 61) can be considered as a cornea apex distance VD.

### <Adjustment Range of Spherical Refractive Power>

In this example, when the variable focus lens 61 and any one of the first spherical lens 51a and the second spherical lens 51b are combined, the range of the spherical refractive power that can be adjusted by only the variable focus lens 61 can be expanded. For example, by setting the variable focus lens 61 to any one of 0 D to -5.00 D and combining the variable focus lens 61 with the first spherical lens 51a (-10.00 D), a spherical refractive power of - 10.00 D to -15.00 D can be generated. For example, by setting the variable focus lens 61 to any one of 0 D to +5.00 D and combining the variable focus lens 61 with the second spherical lens 51b (+10.00 D), a spherical refractive power of +10.00 D to +15.00 D can be generated.

That is, in this example, a spherical refractive power of -5.00 D to +5.00 D can be seamlessly generated using only the variable focus lens. In addition to the variable focus lens 61, by arranging the first spherical lens 51a and the second spherical lens 51b in the examination window 43, a spherical refractive power of -10.00 D to -15.00 D and a spherical refractive power of +10.00 D to +15.00 D can be seamlessly generated. Therefore, the spherical refractive power of -15.00 D to +15.00 D can be seamlessly generated as a whole.

For example, the spherical correction amount of the subject eye E, the spherical refractive power of the variable focus lens 61, and the spherical refractive powers of the first spherical lens 51a and the second spherical lens 51b may be associated in advance. For example, these may be associated in advance such that the spherical refractive power may be generated in the first spherical lens 51a and the second spherical lens 51b (in other words, the first spherical lens 51a or the second spherical lens 51b is arranged) when the spherical correction amount of the subject eye E exceeds the spherical refractive power that can be adjusted by the variable focus lens 61. As an example, a reference table or the like for referring to a value of the spherical refractive power of the variable focus lens 61 and values of the spherical refractive powers of the first spherical lens 51a and the second spherical lens 51b may be prepared in advance based on the spherical correction amount of the subject eye E and stored in a memory 75.

### <Reproduction of Arrangement of Cross Cylinder Lens>

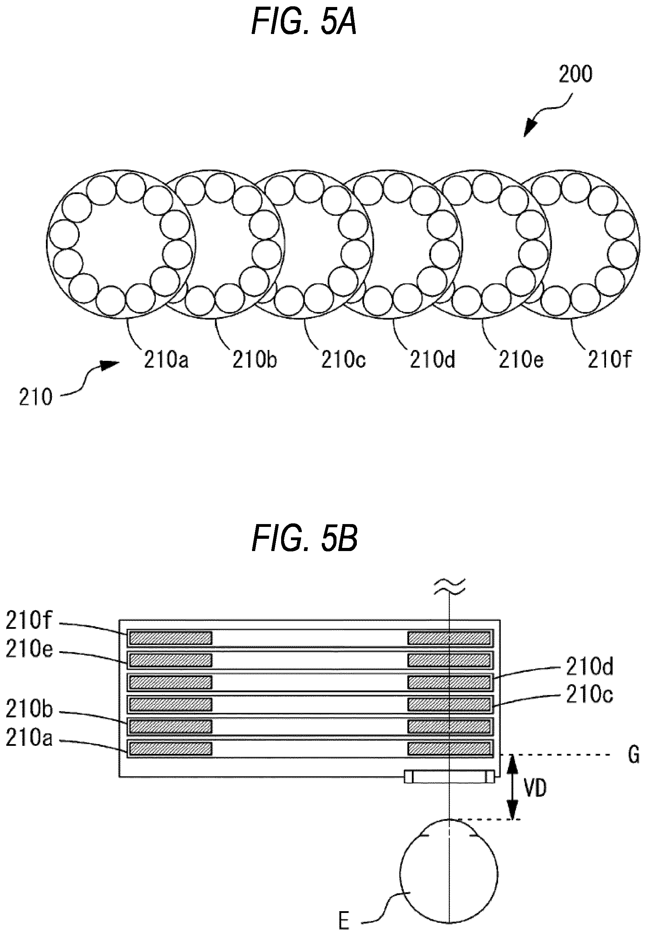

Here, a lens unit of an eye refractive power measurement unit in the related art will be briefly described. FIGS. 5A and 5B are schematic diagrams of a lens unit 200 in the related art. FIG. 5A is an internal configuration diagram of the lens unit 200 in the related art. FIG. 5B is a horizontal sectional view of the lens unit 200 in the related art. In FIGS. 5A and 5B, only a left lens unit is illustrated, and the illustration of a right lens unit is omitted.

The lens unit 200 includes a plurality of lens disks 210. For example, a strong spherical lens disk 210a, a weak spherical lens disk 210b, a strong cylindrical lens disk 210c, a weak cylindrical lens disk 210d, a first auxiliary lens disk 210e, and a second auxiliary lens disk 210f are provided in a direction away from the subject eye E. The strong spherical lens disk 210a is provided with spherical lenses of -18.00 D to +15.00 D at intervals of 3.00 D. The weak spherical lens disk 210b is provided with spherical lenses of -1.00 D to +1.75 D at intervals of 0.25 D. The strong cylindrical lens disk 210c is provided with cylindrical lenses of -1.50 D to -7.50 D at intervals of 1.50 D in terms of the spectacle wearing position. The weak cylindrical lens disk 210d is provided with cylindrical lenses of -0.25 D to -1.25 D at intervals of 0.25 D in terms of the spectacle wearing position. The first auxiliary lens disk 210e is provided with a polarizing filter, a red filter/green filter, a dispersing prism, a Maddox lens, and the like. The second auxiliary lens disk 210f is provided with a plain lens, a rotary prism, an auto-cross cylinder lens, a cross cylinder lens, and the like. For details of the lens unit 200, for example, refer to JP2007-125125A.

The lens unit 200 in the related art is provided with the strong cylindrical lens disk 210c, the weak cylindrical lens disk 210d, and the cross cylinder lens. For example, the cross cylinder lens is a lens in which cylindrical lenses having the same cylindrical refractive power but with opposite signs are combined so that astigmatic axis angles thereof are orthogonal to each other. As an example, the cross cylinder lens is a lens in which cylindrical lenses having -0.25 D and +0.25 D are combined so that astigmatic axis angles thereof are orthogonal to each other. On the other hand, the lens unit 42 in this example has the Stokes lens 62 (the cylindrical lens 62a and the cylindrical lens 62b) instead of the strong cylindrical lens disk 210c and the weak cylindrical lens disk 210d, and has no cross cylinder lens.

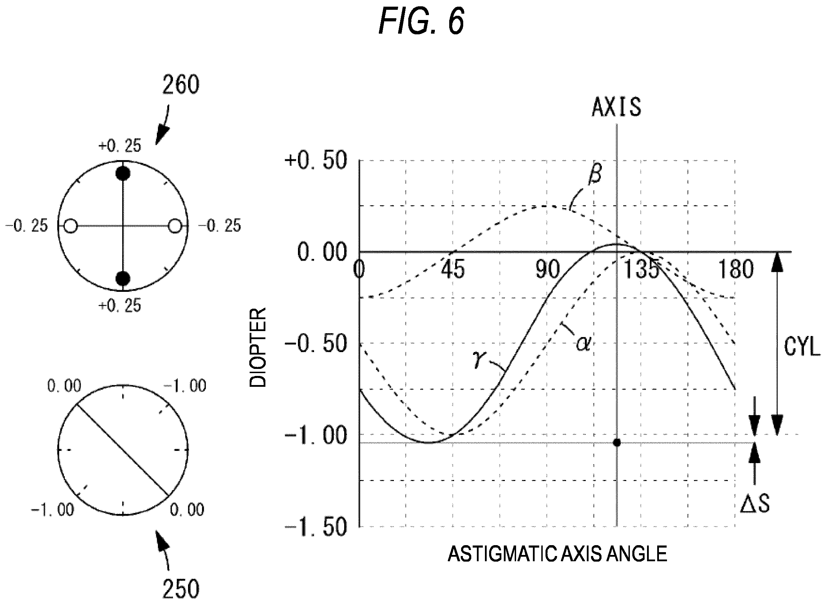

FIG. 6 illustrates a change in a synthetic refractive power of a cylindrical lens of a cylindrical lens disk and a cross cylinder lens in the lens unit 200 in the related art. For example, a cylindrical lens 250 of the weak cylindrical lens disk 210d is switched and arranged in front of the subject eye E. As an example, the cylindrical lens 250 is a lens having a cylindrical refractive power of -1.00 D, and is rotated such that an astigmatic axis angle thereof becomes 135 degrees. At this time, the refractive power of the cylindrical lens 250 varies according to a sine curve α that passes through -0.50 D at 0 degrees, 90 degrees, and 180 degrees, -1.00 D at 45 degrees, and 0.00 D at 135 degrees.

For example, the cross cylinder lens 260 of the first auxiliary lens disk 210e is switched and arranged in front of the subject eye E. As an example, the cross cylinder lens 260 is rotated such that +0.25 D is at 90 degrees. At this time, the refractive power of the cross cylinder lens 260 varies according to a sine curve β that passes through -0.25 D at 0 degrees and 180 degrees, 0.00 D at 45 degrees and 135 degrees, and +0.25 D at 90 degrees.

For example, when both the cylindrical lens 250 and the cross cylinder lens 260 are switched and arranged in front of the subject eye E, a synthetic refractive power obtained by synthesizing the respective refractive powers is generated. At this time, the synthetic refractive power varies according to a sine curve γ that passes through -0.75 D, -1.00 D, -0.25 D, 0.00 D, and -0.75 D in the order of 0 degrees, 45 degrees, 90 degrees, 135 degrees, and 180 degrees. For example, the sine curve α of the refractive power of the cylindrical lens 250 has a vertex at 135 degrees, whereas the sine curve γ of the synthetic refractive power of the cylindrical lens 250 and the cross cylinder lens 260 has a vertex shifted to 122 degrees.

In this example, in consideration of the deviation amount of the astigmatic axis angle that occurs when the arrangement of the cylindrical lens 250 and the cross cylinder lens 260 is assumed, the synthetic axis angle of the cylindrical lens 62a and the cylindrical lens 62b in the Stokes lens 62 is set, and thus the state in which the cylindrical lens 250 and the cross cylinder lens 260 are arranged can be created using only the Stokes lens 62. For example, the arrangement of the cylindrical lens 250 and the cross cylinder lens 260 can be reproduced by adjusting the difference in the axis angle between the cylindrical lens 62a and the cylindrical lens 62b to a difference at which a cylindrical refractive power of -1.00 D is generated, and arranging the synthetic axis angle of the cylindrical lens 62a and the cylindrical lens 62b in accordance with the position of 122 degrees instead of 135 degrees.

For example, the cylindrical correction amount and the astigmatic axis angle of the subject eye E and the synthetic axis angle of the cylindrical lens 62a and the cylindrical lens 62b may be associated in advance. As an example, a reference table for referring to the values of the astigmatic axis angles of the cylindrical lens 62a and the cylindrical lens 62b may be prepared in advance based on the cylindrical correction amount and the astigmatic axis correction amount of the subject eye E and stored in the memory 75.

In the lens unit 200 in the related art, in a state in which the spherical lens, the cylindrical lens 250, and the cross cylinder lens 260 are arranged in front of the subject eye E, an equivalent spherical value of the subject eye E is considered. For example, when the cylindrical lens 250 is switched to change the cylindrical refractive power by 0.25 D (one step), a spherical refractive power of 0.125 D is generated. For example, in a case where the spherical lenses are provided at intervals of 0.25 D, when the cylindrical refractive power is changed by 0.50 D (two steps), the spherical lens can be switched to change the spherical refractive power by 0.25 D, and the compensation in consideration of the equivalent spherical value is performed. In this example, by using the variable focus lens 61, it is possible to easily perform compensation in consideration of a change in the spherical refractive power due to a change in the cylindrical refractive power. When the cylindrical refractive power is finely changed by the Stokes lens 62, the spherical refractive power is changed variously. However, the compensation can be easily carried out even in such a case.

### <Control Unit>

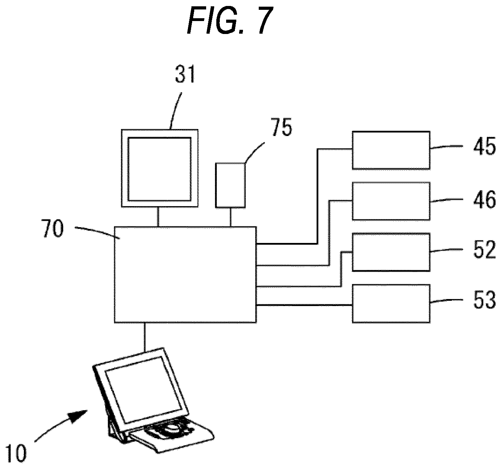

FIG. 7 is a schematic diagram of a control system of the subjective optometry device 100. For example, the control unit 70 includes a CPU (a processor), a RAM, a ROM, and the like. The CPU controls driving of each unit in the subjective optometry device 100. Various types of information are temporarily stored in the RAM. The ROM stores various programs executed by the CPU. The control unit 70 may be implemented by a plurality of control units (that is, a plurality of processors).

The control unit 70 is connected to the display 31, the examiner controller 10, and a non-volatile memory 75 (hereinafter referred to as a memory 75). Further, the control unit 70 is connected to a drive unit of the holding unit 4, a drive unit of the distant-near switching unit 34, a drive unit (drive units 45, 46, 52, and 53) of the eye refractive power measurement unit 40, and the like.

The memory 75 is a non-transitory storage medium capable of storing storage contents even when a supply of power is cut off. For example, the memory 75 may be a hard disk drive, a flash ROM, a USB memory, or the like.

### <Control Operation>

A control operation of the subjective optometry device 100 will be described.

The examiner operates a forehead rest adjustment knob (not shown) to adjust a position of the forehead rest 170 so that the cornea apex distance VD of the subject eye E becomes a predetermined distance (for example, 12 mm). The examiner operates the examiner controller 10 to input the inter-pupillary distance of the subject eye. The control unit 70 adjusts an interval between the lens units 42 to match the examination window 43 to the interpupillary distance.

### <Setting of Initial Correction Amount>

The examiner operates the examiner controller 10 to input an objective eye refractive power (an objective value) acquired in advance in the objective measurement of the subject eye E as an initial correction amount of the subject eye E. That is, an initial spherical correction amount, an initial cylindrical correction amount, and an initial astigmatic axis correction amount are input. The control unit 70 applies an applied voltage to the variable focus lens 61 based on the above-mentioned reference table, and rotates the Stokes lens 62 and the lens disk 50. The variable focus lens 61, the Stokes lens 62, and the lens disk 50 may be controlled in sequence or substantially simultaneously.

In this example, an example is given in which the objective eye refractive power (the objective value) of the subject eye E is a spherical eye refractive power of -6.50 D, a cylindrical eye refractive power of -1.00 D, and an astigmatic axis angle of 135 degrees. At this time, the spherical correction amount of -6.50 D, the cylindrical correction amount of - 1.00 D, and the astigmatic axis correction amount of 135 degrees may be input as the initial correction amount of the subject eye E. For example, the control unit 70 adjusts the spherical refractive power of the variable focus lens 61 to +3.50 D. For example, the control unit 70 changes the difference in the axis angles between the cylindrical lens 62a and the cylindrical lens 62b to adjust the cylindrical refractive power to -1.00 D. For example, the control unit 70 adjusts the synthetic axis angle of the cylindrical lens 62a and the cylindrical lens 62b to 135 degrees. Further, for example, the control unit 70 arranges an opening of the first auxiliary lens disk 50a and the first spherical lens 51a (the spherical refractive power of -10.00 D) of the second auxiliary lens disk 50b in the examination window 43.

Accordingly, the target light flux from the display 31 is corrected to converge on a retina of the subject eye E. That is, the subject eye E is corrected with the spherical correction amount of -6.50 obtained by synthesizing the variable focus lens 61 and the first spherical lens 51a. Further, the subject eye E is corrected with the cylindrical correction amount of -1.0 D and the astigmatic axis correction amount of 135 degrees by the Stokes lens 62.

### <Distant Vision Examination>

When the subject eye E is corrected with the initial correction amount, the examiner operates the examiner controller 10 to start a distant vision examination at a predetermined distance (here, a distant vision examination distance) of the subject eye E. The control unit 70 switches the display 31 to the distant vision arrangement. In addition, the control unit 70 causes the display 31 to display a Landolt ring visual target having a predetermined vision value as an initial visual target. As an example, a Landolt ring visual target having a vision value of 0.8 is displayed as an initial visual target.

The examiner operates the examiner controller 10 to ask the examinee about a direction of a gap in the ring of the Landolt ring visual target while switching the Landolt ring visual target. For example, if the examinee's answer is correct, the vision value of the Landolt ring visual target is switched to a vision value higher by one level. In other words, the increment of the vision value of the Landolt ring visual target is increased by one, and the vision value of the Landolt ring visual target is switched to a value larger than the current value. As an example, the vision value of the Landolt ring visual target is switched from 0.8 to 0.9. For example, if the examinee's answer is incorrect, the vision value of the Landolt ring visual target is switched to a vision value lower by one level. In other words, the increment of the vision value of the Landolt ring visual target is decreased by one, and the vision value of the Landolt ring visual target is switched to a value smaller than the current value. As an example, the vision value of the Landolt ring visual target is switched from 0.8 to 0.7. The examiner repeats the procedures to acquire a maximum vision value of the Landolt ring visual targets that the subject eye E can read.

Subsequently, the examiner operates the examiner controller 10 to ask the examinee about the direction of the gap in the ring of the Landolt ring visual target while switching the correction amount of the subject eye E. For example, if the examinee's answer is correct, the spherical correction amount is switched to a correction amount weaker by one level. That is, the spherical correction amount is reduced by one step and is switched to a value smaller than the current value. As an example, by changing the spherical refractive power of the variable focus lens 61 from +3.50 D to +3.25 D, the spherical correction amount for correcting the subject eye E is switched from -6.50 D to -6.25 D. For example, if the examinee's answer is incorrect, the spherical correction amount is switched to a correction amount stronger by one level. That is, the spherical correction amount is increased by one step and is switched to a value larger than the current value. As an example, by changing the spherical refractive power of the variable focus lens 61 from +3.50 D to +3.75 D, the spherical correction amount is switched from -6.50 D to -6.75 D.

The control unit 70 arranges the first spherical lens 51a and the second spherical lens 51b as necessary in accordance with the spherical correction amount to be applied to the subject eye E. Of course, depending on the examinee's answer, the cylindrical correction amount and the astigmatic axis correction amount may be switched as well as the spherical correction amount. In this example, one step of the spherical correction amount can be changed more finely than -0.25 D by the variable focus lens 61. Similarly, one step of the cylindrical correction amount can be changed more finely than -0.25 D by the cylindrical lens 62a and the cylindrical lens 62b of the Stokes lens 62. The examiner repeats the procedures to acquire a value of a most positive corrected refractive power (that is, a best correction value) at which a maximum visual acuity of the subject eye E is obtained.

### <Addition Power Examination>

When the distant vision examination for the subject eye E is completed, the examiner operates the examiner controller 10 to start an addition power examination in a state in which the subject eye E is corrected with the best correction value at a predetermined distance (a distant vision examination distance). For example, the examiner determines the necessity of addition based on at least any one of an age of the examinee, an accommodative power of the subject eye E, a refractive power of the subject eye E, and the like, and sets an initial addition power. Of course, the necessity of addition and the initial addition power may be automatically determined and set by the control unit 70.

In this example, an example is given in which the best correction value (the subjective value) of the subject eye E is acquired as the spherical correction amount of -6.00 D, the cylindrical correction amount of -1.25 D, and the astigmatic axis correction amount of 135 degrees, and the initial addition power is set to +1.00 D. In the addition power examination using the lens unit 200 in the related art, the subject eye E is corrected with the best correction value, the cross cylinder lens is arranged such that -0.50 D is at 0 degrees and +0.50 D is at 90 degrees, and the initial addition power is increased or decreased. In this example, the subject eye E is corrected with the best correction value, the state in which the cross cylinder lens is arranged is reproduced using the Stokes lens 62, and then the initial addition power is increased or decreased.

First, the control unit 70 corrects the subject eye E with the best correction value to create the first state assuming that the cross cylinder lens is arranged. The control unit 70 assumes that as the cylindrical lens 250 of the lens unit 200 in the related art, a lens with a cylindrical refractive power of -1.25 D is arranged at 135 degrees, and that the cross cylinder lens 270 is arranged such that +0.50 D is at 90 degrees, and rotates the cylindrical lens 62a and the cylindrical lens 62b of the Stokes lens 62 to correct the cylindrical correction amount of the subject eye E to -1.25 D and the astigmatic axis angle to 135 degrees.

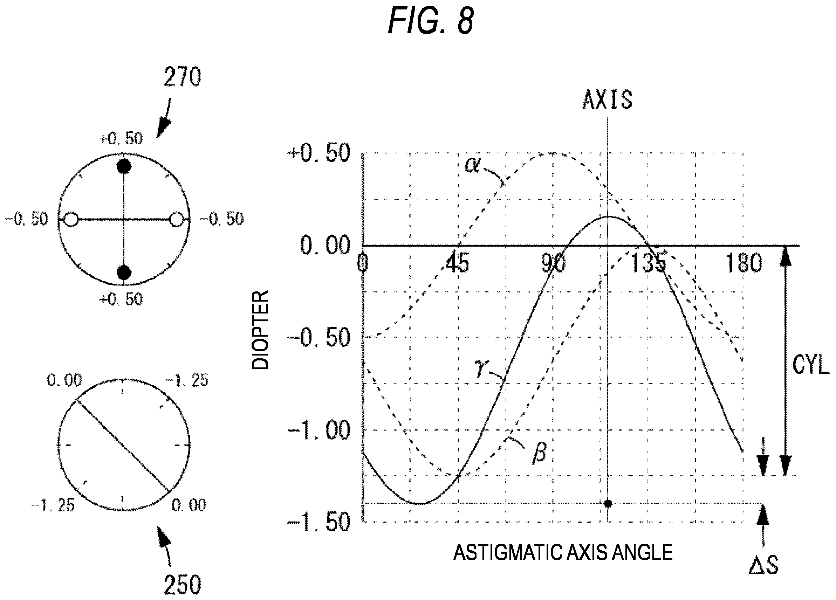

FIG. 8 illustrates a change in the synthetic refractive power of the cylindrical lens 250 and the cross cylinder lens 270. The refractive power of the cylindrical lens 250 varies with the sine curve α passing through -1.25 D at 45 degrees, and the refractive power of the cross cylinder lens 270 varies with the sine curve β passing through +0.50 D at 90 degrees. The sine curve γ of the synthetic refractive power of the cylindrical lens 250 and the cross cylinder lens 270 has a vertex at a position of 116 degrees. Therefore, the control unit 70 adjusts the difference in the axis angle between the cylindrical lens 62a and the cylindrical lens 62b of the Stokes lens 62 to a difference at which a cylindrical refractive power of -1.25 D is generated, and the synthetic axis angle of the cylindrical lens 62a and the cylindrical lens 62b is arranged in accordance with the position of 116 degrees instead of 135 degrees. Accordingly, the subject eye E is corrected by reproducing a state in which the cylindrical correction amount (CYL) of -1.25 D, the astigmatic axis correction amount (AXIS) of 135 degrees, and the cross cylinder lens 270 (+0.50 D in a direction of 90 degrees) are combined.

At this time, a spherical refractive power ΔS is generated due to the deviation amount between the astigmatic axis correction amount of the subject eye E and the synthetic axis angle of the cylindrical lens 62a and the cylindrical lens 62b. The spherical refractive power ΔS is an amount that appears as a refractive power difference between the refractive power of the cylindrical lens 250 and the synthetic refractive power of the cylindrical lens 250 and the cross cylinder lens 260 (in FIG. 8, a difference between vertices of the sine curve α and the sine curve γ in a vertical axis). The spherical refractive power ΔS varies depending on the deviation amount between the astigmatic axis correction amount and the synthetic axis angle described above, but is smaller than one step (0.25 D) and is a slight amount. For example, in this example, the spherical refractive power ΔS is -0.15 D.

The control unit 70 changes the spherical refractive power of the variable focus lens 61 to +4.00 D, and switches and arranges the first spherical lens 51a (-10.00 D) to correct the subject eye E with the spherical correction amount of -6.00 D. However, since the spherical refractive power ΔS is generated by the Stokes lens 62, in practice, the spherical refractive power of the variable focus lens 61 is set to +3.85 D, and the subject eye E is corrected with the spherical correction amount -6.15 D. For example, by using the variable focus lens 61 in this way, it is possible to finely adjust a numerical value in consideration of the spherical refractive power ΔS.

The examiner operates the examiner controller 10 to display a cross grid visual target on the display 31. Further, the examiner operates the examiner controller 10 and presses a switch (not shown) to switch between the first state assuming that the subject eye E is corrected with the best correction value and the cross cylinder lens is arranged, and the second state in which the initial addition power of +1.00 D is further added in the first state. The control unit 70 changes the spherical refractive power of the variable focus lens 61 in response to an operation signal from the switch (not shown). Here, the spherical refractive power of the variable focus lens 61 is changed to +3.85 D or +4.85 D. Accordingly, the spherical correction amount of the subject eye E is switched between -6.00 D (actually, -6.15 D) to which the initial addition power is not added and -5.00 D (actually, -5.15 D) to which the initial addition power is added.

The examiner asks the examinee about how the cross grid visual target looks, and changes the addition power according to the examinee's answer. The control unit 70 changes the spherical refractive power of the variable focus lens 61 according to the spherical refractive power added to the subject eye E, and arranges the first spherical lens 51a and the second spherical lens 51b as necessary. The examiner repeats the procedures, and determines a level at which a vertical line and a horizontal line of the cross grid visual target are equally seen as a proper addition power for the subject eye E.

### <Near Vision Examination>

When the addition power examination for the subject eye E is completed, the examiner operates the examiner controller 10 to start a near vision examination at a predetermined distance (here, a near vision examination distance) of the subject eye E. The control unit 70 switches the display 31 to the near vision arrangement. In the lens unit 200 in the related art, the cross cylinder lens 270 is removed when transitioning from the addition power examination to the near vision examination, and thus, also in this example, the variable focus lens 61 and the Stokes lens 62 (the cylindrical lens 62a and the cylindrical lens 62b) are also adjusted to reproduce the state in which the cross cylinder lens 270 is removed. A maximum vision value is acquired also in the near vision examination, but this procedure is basically the same as that of the distant vision examination, and thus the description thereof is omitted.

As described above, for example, the subjective optometry device in this example controls the first correction means which changes the spherical refractive power of the target light flux using the variable focus member (here, the variable focus lens 61) which has a variable focal length, and the second correction means which switches the optical member (here, the first spherical lens 51a ,the second spherical lens 51b) to arrange another optical member for changing the spherical refractive power of the target light flux, so that the spherical refractive power of the target light flux is changed. For example, the variable focus member can continuously change the spherical refractive power. Therefore, the spherical correction amount of the subject eye can be finely adjusted to appropriately correct the subject eye. However, it is technically difficult for the variable focus member to generate a high spherical refractive power, and there is a possibility that a case where the spherical correction amount of the subject eye is large, or the like cannot be coped only by changing the spherical refractive power by the variable focus member. In this example, by combining the variable focus lens and the first spherical lens or the second spherical lens as necessary, it is possible to generate a high spherical refractive power. Therefore, even when the spherical correction amount of the subject eye is large, the subject eye can be appropriately corrected, and the eye refractive power of the subject eye can be accurately measured.

For example, in the related art, when the spherical lens is switched in front of the subject eye, there are cases where a sudden change from a certain spherical refractive power to another spherical refractive power (in other words, a seam is formed in the spherical refractive power) causes a sense of discomfort, and a sound generated when switching the spherical lens gives a sense of annoyance. In this example, by adjusting the desired spherical refractive power using the variable focus member, the spherical refractive power varies seamlessly (without seams), and thus the measurement can be advanced without a sense of discomfort. In addition, since a switching sound of the spherical lens does not occur, the annoyance can be reduced.

For example, in the subjective optometry device in this example, the first correction means can change the spherical refractive power of the target light flux in the range of the first refractive power, and switch at least the optical member to arrange another optical member, in the case where the changed spherical refractive power obtained by changing the spherical refractive power of the target light flux exceeds the range of the first refractive power of the spherical refractive power in the first correction means. For example, in a state in which the variable focus member is arranged in front of the subject eye, the spherical refractive power can be continuously changed within the range of the first refractive power, and a subjective examination for the subject eye can be carried out smoothly. In addition, for example, in a state in which the variable focus member and the optical member are arranged in front of the subject eye, the spherical refractive power (the synthetic spherical refractive power) can be enlarged by the optical member, the synthetic spherical refractive power can be continuously changed by the variable focus member, and the subjective examination for the subject eye can be carried out smoothly.

For example, in the subjective optometry device in this example, a step in which the first correction means enables to change the spherical refractive power of the target light flux is smaller than a step in which the second correction means enables to change the spherical refractive power of the target light flux. Accordingly, the spherical refractive power of the target light flux can be changed more finely using the variable focus member. Further, even when the variable focus member and the optical member are combined, the spherical refractive power of the target light flux can be changed more finely within the range of the synthetic spherical refractive power.

In addition, for example, in the addition power examination for measuring the addition power to the correction amount at the predetermined examination distance of the subject eye, the subjective optometry device in this example controls the Stokes lens that is independently rotatable in front of the subject eye, and changes the synthetic axis angle of the first cylindrical lens and the second cylindrical lens to a predetermined axis angle based on the cylindrical correction amount and the astigmatic axis correction amount of the subject eye. Here, the synthetic axis angle is an axis angle in consideration of the deviation amount of the astigmatic axis correction amount that occurs when the optical member and the cross cylinder lens are assumed to be arranged in order to correct the subject eye with the cylindrical correction amount and the astigmatic axis correction amount. For example, in a state in which the Stokes lens is arranged in front of the subject eye, it is possible to continuously change the cylindrical refractive power by respectively rotating the two cylindrical lenses to adjust the difference in the axis angle. By integrally rotating the two cylindrical lenses to adjust the synthetic axis angle, it is possible to continuously change the astigmatic axis angle. At this time, in this example, since the deviation amount of the astigmatic axis correction amount caused by the Stokes lens is taken into consideration, the cylindrical correction amount and the astigmatic axis correction amount of the subject eye can be appropriately corrected, and the eye refractive power of the subject eye can be accurately measured.

For example, in the related art, when the cylindrical lens is switched in front of the subject eye, as in the case of switching the spherical lens described above, the change in the cylindrical refractive power in a state in which a seam is formed causes a sense of discomfort, and a switching sound of the cylindrical lens gives a sense of annoyance. However, in this example, by adjusting the desired cylindrical refractive power using the Stokes lens, the cylindrical refractive power varies seamlessly (without seams), and no switching sound is generated, and thus it is possible to reduce the sense of discomfort and annoyance.

For example, the subjective optometry device in this example compensates the deviation amount of the spherical correction amount of the subject eye, that is, the deviation amount of the spherical correction amount caused by aligning the first cylindrical lens and the second cylindrical lens with the synthetic axis angle, by arranging the compensation optical member in front of the subject eye. Accordingly, even when the cylindrical refractive power and the astigmatic axis angle of the target light flux are changed by the Stokes lens and the cylindrical correction amount and the astigmatic axis correction amount of the subject eye are adjusted, the deviation amount of the spherical correction amount of the subject eye is appropriately corrected by the compensation optical member, and thus the eye refractive power of the subject eye can be measured accurately.

For example, in the subjective optometry device in this example, the compensation optical member for compensating the deviation amount of the spherical correction amount of the subject eye is a variable focus member (here, the variable focus lens 61) having a variable focal length, and compensates the deviation amount of the spherical correction amount of the subject eye by changing the spherical refractive power of the variable focus member. For example, the deviation amount of the spherical correction amount caused by the first cylindrical lens and the second cylindrical lens can be changed with a value smaller than the interval (0.25 D) of the plurality of spherical lenses configured by the lens unit in the related art. Therefore, when a spherical optical member having a fixed focal length is used as the compensation optical member, there is a possibility that the spherical optical member cannot fully cope with the deviation amount of the spherical correction amount. However, by using the variable focus member which has a variable focal length, it is possible to finely cope with the deviation amount of the spherical correction amount. As a result, the subject eye can be appropriately corrected with a desired spherical correction amount.

For example, the subjective optometry device in this example acquires the addition power of the subject eye, and switches between the first state in which the first cylindrical lens and the second cylindrical lens are aligned with the synthetic axis angle and the second state in which the correction optical member based on the addition power is arranged in the first state. That is, the subject eye is corrected with a predetermined spherical correction amount, a predetermined cylindrical correction amount, and a predetermined astigmatic axis correction amount, and the first state in which the addition power is not added and the second state in which the addition power is added are switched in a state in which the deviation amount of the spherical correction amount is further compensated. Accordingly, it is possible to accurately measure an optimal addition power for the subject eye.

For example, in the subjective optometry device in this example, the compensation optical member for compensating the deviation amount of the spherical correction amount of the subject eye and the correction optical member for correcting the subject eye with the spherical correction amount are used as both. Accordingly, it is possible to easily adjust the spherical correction amount for correcting the subject eye in consideration of the deviation amount of the spherical correction amount of the subject eye.

### <Modification>

In the subjective optometry device in this example, the configuration in which the spherical refractive power is adjusted in steps of 0.25 D per step has been described as an example, but the present invention is not limited thereto. For example, the subjective optometry device in this example can continuously change the spherical refractive power of the variable focus lens 61. Therefore, one step of the spherical refractive power may be adjusted with a value smaller than 0.25 D (for example, 0.10 D, 0.05 D, or the like). Similarly, in the subjective optometry device in this example, the configuration in which the cylindrical refractive power is adjusted in steps of 0.25 D per step has been described as an example, but the present invention is not limited thereto. For example, the subjective optometry device in this example can continuously change the cylindrical refractive power of the Stokes lens 62. Therefore, one step of the cylindrical refractive power may be adjusted with a value smaller than 0.25 D (for example, 0.10 D, 0.05 D, or the like).

In the subjective optometry device in this example, the configuration in which the spherical correction amount of the subject eye E, the spherical refractive power of the variable focus lens 61, and the arrangement of the first spherical lens 51a and the second spherical lens 51b are associated in advance has been described as an example, but the present invention is not limited thereto. For example, the subjective optometry device in this example may be configured to determine whether or not to arrange the first spherical lens 51a and the second spherical lens 51b, based on the spherical correction amount of the subject eye E. In this case, the control unit 70 may determine whether or not to arrange the first spherical lens 51a and the second spherical lens 51b, based on whether or not the spherical correction amount of the subject eye E exceeds a predetermined threshold value. For example, the predetermined threshold value may be a maximum value or a minimum value based on a range of the spherical refractive power that can be adjusted by the variable focus lens 61.

In this way, the subjective optometry device in this example determines whether or not the optical member (here, the first spherical lens 51a and the second spherical lens 51b) is arranged in front of the subject eye E, based on the spherical correction amount of the subject eye (that is, the changed spherical refractive power obtained by changing the spherical refractive power of the target light flux), and switches at least the optical member to arrange another optical member based on a determination result. Accordingly, a case that cannot be coped only by adjusting the spherical refractive power of the variable focus member (here, the variable focus lens 61), such as a case where the spherical correction amount of the subject eye is large, can be easily grasped, and the variable focus member and the optical member are combined to accurately measure the eye refractive power of the subject eye.

For example, the subjective optometry device in this example determines whether or not the optical member is arranged in front of the subject eye, based on the spherical correction amount of the subject eye (the changed spherical refractive power of the target light flux) and the range of the refractive power in the correction means. Accordingly, when it is not possible to cope by only the variable optical member, it is possible to appropriately arrange the optical member to accurately measure the eye refractive power of the subject eye.

In the subjective optometry device in this example, in some cases, the correction amount is further changed from the initial correction amount of the subject eye E or the correction amount switched after the initial correction amount to a different correction amount in the distant vision examination and the near vision examination. At this time, the control unit 70 may change whether to control only the variable focus member 61 or to control both the variable focus member 61 and the first spherical lens 51a (or the second spherical lens 51b) depending on a change amount in the two correction amounts. For example, when the change amount in the two correction amounts does not exceed a predetermined change amount, the control unit 70 may control only the variable focus member 61. For example, when the change amount in the two correction amounts exceeds the predetermined change amount, the control unit 70 may control both the variable focus member 61 and the first spherical lens 51a (the second spherical lens 51b). For example, the change amount in the two correction amounts may be set in advance and may be, for example, ±3.00 D.

In this way, when the spherical correction amount of the subject eye (the changed spherical refractive power obtained by changing the spherical refractive power of the target light flux) is adjusted from the first changed spherical refractive power to the second changed spherical refractive power different from the first changed spherical refractive power, the subjective optometry device in this example controls only the first correction means according to a change amount between the first changed spherical refractive power and the second changed spherical refractive power, to change the focal length of the variable focus member. Accordingly, for example, it is possible to easily adjust a spherical correction amount necessary for the correction of the subject eye and smoothly measure the eye refractive power of the subject eye.

In the subjective optometry device in this example, after the correction amount is switched from the initial correction amount to the different correction amount for the subject eye E, the subjective value (for example, the best correction value) is acquired by controlling only the variable focus member 61. In this case, it may be set whether or not the first spherical lens 51a or the second spherical lens 51b is arranged in the examination window 43 in advance according to the initial correction amount of the subject eye E. For example, when the initial correction amount of the subject eye E is the spherical correction amount of -1.00 D, -1.00 D may be generated using only the variable focus member 61 (the spherical refractive power of -5.00 D to +5.00 D). For example, when the initial correction amount of the subject eye E is the spherical correction amount of -4.50 D, -4.5 D may be generated by combining the variable focus member 61 and the first spherical lens 51a (the spherical refractive power of -10.00 D). For example, a change amount until the initial correction amount of the subject eye E is adjusted to a final correction amount (that is, a difference between the initial correction amount and the final correction amount) can be roughly grasped from experiments, simulations, and the like. Therefore, by arranging the first spherical lens 51a in advance according to the initial correction amount of the subject eye E, it is not necessary to rotate the second auxiliary lens disk 50b during the measurement of the subject eye E, and it is possible to perform more seamless handling. In such a configuration, a plurality of spherical lenses may be provided, and the spherical refractive power thereof may be changed with a predetermined step (for example, in units of 3.00 D or the like).

### REFERENCE SIGNS LIST

1 housing
2 presentation window
10 examiner controller
30 light projecting optical system
40 eye refractive power measurement unit
43 examination window
60 control unit
100 subjective optometry device

## Claims

1. A subjective optometry device for subjectively measuring an eye refractive power of a subject eye, the subjective optometry device comprising:
a correction means arranged in front of the subject eye, and configured to change an optical characteristic of a target light flux emitted from a target presentation means; and
a control means configured to control the correction means,
wherein the correction means includes a first correction means configured to change a spherical refractive power of the target light flux using a variable focus member which has a variable focal length, and a second correction means configured to switch an optical member to arrange another optical member for changing the spherical refractive power of the target light flux, and
the control means controls the first correction means and the second correction means to change the spherical refractive power of the target light flux.

2. The subjective optometry device according to claim 1,
wherein the first correction means is configured to change the spherical refractive power of the target light flux in a range of a first refractive power, and
the control means controls at least the second correction means to switch the optical member to arrange another optical member, in a case where a changed spherical refractive power obtained by changing the spherical refractive power of the target light flux exceeds the range of the first refractive power in the first correction means.

3. The subjective optometry device according to claim 2, further comprising:
a determination means configured to determine whether or not to arrange the optical member, based on the changed spherical refractive power of the target light flux,
wherein the control means controls at least the second correction means based on a determination result of the determination means such that the optical member is switched to arrange another optical member.

4. The subjective optometry device according to claim 3,
wherein the determination means determines whether or not to switch the optical member to arrange another optical member, based on the changed spherical refractive power of the target light flux and the range of the first refractive power in the first correction means.

5. The subjective optometry device according to any one of claims 1 to 4,
wherein a step in which the first correction means enables to change the spherical refractive power of the target light flux is smaller than a step in which the second correction means enables to change the spherical refractive power of the target light flux.

6. The subjective optometry device according to any one of claims 1 to 5,
wherein in a case where the changed spherical refractive power of the target light flux is adjusted from a first changed spherical refractive power to a second changed spherical refractive power different from the first changed spherical refractive power, the control means controls only the first correction means according to a change amount between the first changed spherical refractive power and the second changed spherical refractive power, to change the focal length of the variable focus member.

7. A subjective optometry program used in a subjective optometry device for subjectively measuring an eye refractive power of a subject eye, the subjective optometry device including a correction means that is arranged in front of the subject eye and configured to change an optical characteristic of a target light flux emitted from a target presentation means, and includes a first correction means configured to change a spherical refractive power of the target light flux using a variable focus member which has a variable focal length, and a second correction means configured to switch an optical member to arrange another optical member for changing the spherical refractive power of the target light flux,
the subjective optometry program comprising an instruction which, when executed by a processor of the subjective optometry device, causes the subjective optometry device to perform:
a control step of controlling the correction means,
wherein in the control step, the first correction means and the second correction means are controlled to change the spherical refractive power of the target light flux.
